(19) Europäisches Patentamt / European Patent Office / Office européen des brevets

(11) **EP 2 711 676 B1**

(12) **EUROPÄISCHE PATENTSCHRIFT**

(45) Veröffentlichungstag und Bekanntmachung des Hinweises auf die Patenterteilung:
**07.10.2020 Patentblatt 2020/41**

(51) Int Cl.:
*G01L 1/24* *(2006.01)*        *A61M 25/00* *(2006.01)*
*A61B 5/00* *(2006.01)*        *A61B 18/14* *(2006.01)*

(21) Anmeldenummer: **13180501.2**

(22) Anmeldetag: **15.08.2013**

(54) **Faseroptischer Kraftsensor, Kraftmesseinrichtung und Katheter**

Fiber-optic force sensor, force measurement device and catheter

Capteur de force à fibre optique, dispositif de mesure de force et cathéter

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.09.2012 US 201261703272 P**

(43) Veröffentlichungstag der Anmeldung:
**26.03.2014 Patentblatt 2014/13**

(73) Patentinhaber: VascoMed GmbH
**79589 Binzen (DE)**

(72) Erfinder:
• Fandrey, Stephan
  **8910 Affoltern am Albis (CH)**
• Bitzer, Andreas
  **8032 Zürich (CH)**

(74) Vertreter: **Galander, Marcus et al
Biotronik Corporate Services SE
Corporate Intellectual Property
Sieversufer 7 - 9
12359 Berlin (DE)**

(56) Entgegenhaltungen:
**US-A1- 2009 177 095     US-A1- 2012 220 879**

EP 2 711 676 B1

**Beschreibung**

Die Erfindung betrifft einen faseroptischen Kraftsensor, der eine in einem Sensorhalter gehalterter FBG-Faser mit mindestens einem Kraftsensorbereich umfasst. Sie betrifft des Weiteren einen Katheter, insbesondere Ablationskatheter, mit einem in einen distalen Abschnitt integrierten Kraftsensor, der zur Messung des Betrages und der Richtung einer auf den distalen Abschnitt wirkenden äußeren Kraft ausgebildet und angeordnet ist, wobei der Kraftsensor durch einen Kraftsensorbereich auf einer FBG-Faser gebildet ist.

In bestimmten Einsatzfeldern von Kathetern oder ähnlichen Vorrichtungen, z.B. Elektrodenleitungen, ist eine Andruckkraft an benachbartes Gewebe von Bedeutung für deren Funktion, sodass eine Erfassung dieser Kontaktkraft von Interesse ist. Dies trifft in besonderem Maße für sogenannte Ablationskatheter zu, mit denen Gewebebereiche verödet bzw. Gewebeteile abgetragen werden.

Es ist ein Ablationskatheter ("TactiCath", Fa. Endosense) bekannt, welches die Messung einer auf das distale Katheterende einwirkenden Kraft - im Anwendungsfall also der erwähnten Kontaktkraft - nach Betrag und Richtung während eines Ablationsvorganges ermöglicht. Dieser Katheter nutzt das Prinzip des sogenannten FBG(Faser-Bragg-Gitter)-Sensors, wobei drei Fasern mit jeweils einem FBG-Sensor am Faserende die für eine 3D-Kraftmessung benötigte Gruppe von Sensoren bilden, die zur gemeinsamen Messsignalverarbeitung an eine Signalverarbeitungseinheit einschließbar sind. Die Sensoren sind in einem Winkelabstand von 120° außen auf einem verformbaren Zylinder aufgebracht.

In der US 2008/0285909 A1 wird die Funktionsweise von FBG-Sensoren zur Bestimmung von Verdrillungen oder Krümmungen des Katheterkörpers ausführlich beschrieben, und auch die Funktionsweise des vorgenannten Kraftsensors mit mehreren FBG-Fasern auf einem verformbaren Zylinder ist in dieser Druckschrift erläutert.

Es ist, wie in der WO 2009/138957 A2 beschrieben, eine Temperaturkompensation mittels dreier elektrischer Thermoelemente vorgesehen, weil bei dem FBG-Messverfahren bereits kleine Temperaturänderungen bzw. Abweichungen zwischen den einzelnen Sensoren große Messunsicherheiten verursachen können und bei einem elektrothermischen Ablationsvorgang ganz erhebliche Temperaturschwankungen an der Spitze des Ablationskatheters auftreten können.

Das optische Messprinzip der FBG-Sensorik ist allgemein sowie insbesondere auch in seiner Anwendung für Kraftmessungen und Temperaturmessungen bekannt; vgl. etwa www.wikipedia.org/wiki/Fiber_Bragg_grating oder A. Othonos, K. Kalli:"Fiber Bragg Gratings: Fundamentels and Applications in Telecommunications and Sensing" Artec House 1999, sowie (speziell bezogen auf Spannungs- und Temperaturmessungen) US 5,399,854. Eine ausführliche Erläuterung des Messprinzips ist daher hier nicht erforderlich.

Unabhängig von diesem Messprinzip sind auch andere Lösungen für eine Kontaktkraftmessung an einem Führungsdraht oder Katheter bekannt, bspw. unter Einsatz eines optischen Sensors, wie in der WO 2009/007857 A2 beschrieben, oder unter Einsatz eines Halbleitersensors an der Spitze eines Führungsdrahtes, wie in der WO 2008/003307 A2 beschrieben. Eine jüngere Lösung ist Gegenstand der auf die Anmelderin zurückgehenden US 2012/0220879 A1.

Das Dokument US 2009/0177095 A1 offenbart einen Katheter mit einer optischen Kraftmesseinrichtung. An einem Halteelement mit mehreren Segmenten sind mehrere optische Fasern angeordnet. Bei einer Krafteinwirkung verschieben sich die Segmente, wobei die Verschiebung mittel Sensoren in den optischen Fasern bestimmt und hieraus die Kraft ermittelt wird.

Im Hinblick auf den o. g. Stand der Technik besteht eine Aufgabe darin, einen für einfache Anwendungen geeigneten Kraftsensor mit besonders einfacher Signalverarbeitung anzugeben. Eine weitere Aufgabe besteht darin, eine gleichfalls hinsichtlich ihrer Signalverarbeitung vereinfachte Kraftmesseinrichtung anzugeben, die für eine mehrachsige Kraft- bzw. Druckerfassung und entsprechende Anwendungen geeignet ist. Eine weitere Aufgabe besteht darin, einen einfach aufgebauten und somit kostengünstig zu realisierenden Katheter für spezielle Anwendungen anzugeben, bei dessen Einsatz die Patientensicherheit in hohem Maße gewährleistet ist.

In ihrem den Kraftsensor betreffenden Aspekt wird die Aufgabenstellung durch einen Kraftsensor mit den Merkmalen des Anspruchs 1 gelöst. In ihrem den Katheter betreffenden Aspekt wird die Aufgabenstellung durch einen Katheter mit den Merkmalen des Anspruchs 6 gelöst. Zweckmäßige Fortbildung des Erfindungsgedankens ist Gegenstand der jeweiligen abhängigen Ansprüche.

Die Erfindung geht von der Erkenntnis aus, dass bei dem vorbekannten, konstruktiv einfachen und mit geringen Abmessungen realisierbaren Ein-Faser-Kraftsensor mit jedem Sensorbereich (abhängig von dessen Winkellage auf der FBG-Faser) jeweils eine spezielle Kombination aus den in den drei Raumrichtungen wirkenden Kraftkomponenten gemessen wird. Dabei ist die Sensitivität in z-Richtung immer deutlich geringer, verglichen mit seitlichen Lasten aus der xy-Richtung aufgrund der Biegemomente. Bei dieser Konstruktion ist es nicht ohne Weiteres möglich, die Sensitivität der z-Richtung zu erhöhen, ohne die Sensitivität in xy-Richtung zu erhöhen, wodurch der Sensor ab einem gewissen Punkt zu instabil wird. Damit lässt sich der Dynamikbereich der FBG-Sensoren für die mechanischen Lasten nicht optimal ausnutzen, so dass Temperatureinflüsse in dem System immer in der Größenordnung der mechanischen Beanspruchung liegen. Daher sind zusätzliche Sensorbereiche notwendig, um eine geeignete Temperaturkompensation zu erreichen, wodurch sich die Komplexität und die Kosten eines solchen Systems erhöhen.

Aufbauend auf dieser Erkenntnis, gehört zur Erfindung die Überlegung einen im Wesentlichen nur in z-Richtung sensitiven Kraftsensor der erwähnten Art zu bauen sowie - diese Idee weiterführend - eine Kraftmesseinrichtung zur realisieren, die eine solche, im Wesentlichen in z-Richtung sensitive Sensorkomponente umfasst. Demgemäß wird vorgeschlagen, dass die FBG-Faser einen Kraftsensorbereich umfasst, dem Versteifungsmittel zur Unterdrückung von lateral einwirkenden Kräften und Biegemomenten derart zugeordnet sind, dass der Kraftsensorbereich ein vorherrschend einachsiges Ansprechverhalten zur Erfassung nur des Betrages einer axial in Faserrichtung wirkenden Kraft hat.

Gemäß einem weiteren Aspekt der Erfindung sind in dem Sensorhalter einer FBG-Faser, welche drei Kraftsensorbereiche umfasst, einem der drei Kraftsensorbereiche Versteifungsmittel zur Unterdrückung von lateral einwirkenden Kräften und Biegemomenten derart zugeordnet, dass der Kraftsensor ein vorherrschend einachsiges Ansprechverhalten zur Erfassung nur des Betrages einer axial in Faserrichtung wirkenden Kraft hat. Gemäß dem letztgenannten, eine variable einsetzbare Kraftmesseinrichtung betreffenden Aspekt sind die verbleibenden beiden Kraftsensorbereiche auf der FBG-Faser, insbesondere um 90°, winkelversetzt angeordnet, derart, dass sie zur Erfassung des Betrages und der Richtung der Projektion der einwirkenden Kraft auf die Ebene senkrecht zur Erstreckung der FBG-Faser ausgebildet sind.

Die FBG-Faser ist auf einem in axialer Richtung abschnittsweise nachgiebigen und biegeweichen Sensorhalter angeordnet, dem ein axial gegenüber dem Sensorhalter verschiebliches, aber lateral unverschiebliches Versteifungsteil zugeordnet ist. In einer Ausgestaltung dieser Ausführung ist der Sensorhalter durch ein abschnittsweise weiches Außenrohr und das Versteifungsteil durch ein hartes Innenrohr oder der Sensorhalter durch ein abschnittsweise weiches Innenrohr und das Versteifungsteil durch ein hartes Außenrohr gebildet, wobei das Innenrohr im Außenrohr axial verschieblich geführt ist.

In einer hierzu alternativen Ausführung ist die FBG-Faser auf einem Sensorhalter angeordnet, der im Bereich des Kraftsensorbereiches mit einachsigem Ansprechverhalten in axialer Richtung weich, aber durch mindestens zwei gegenüber der Längsachse des Sensorhalters geneigte, insbesondere einen Winkel zwischen 45° und 90° mit der Längsachse einschließende, Querstreben gegenüber lateral einwirkenden Kräften und Biegemomenten ersteift ist. Eine Ausgestaltung dieser Ausführung sieht vor, dass der Sensorhalter genau zwei Querstreben in räumlicher Zuordnung zum Kraftsensorbereich aufweist, die an beiden Enden durch Längsstreben verbunden sind, wobei der Sensorhalter, in axialer Richtung gesehen, vor der ersten und hinter der zweiten Querstrebe je einen in axialer Richtung verformbaren Abschnitt hat.

Entsprechende Ausführungen gelten auch für die erwähnte Kraftmesseinrichtung, und zwar speziell bezüglich des Kraftsensorbereichs mit vorherrschend einachsigem Ansprechverhalten. Diese Ausführungen werden hier nicht wiederholt; es wird jedoch angemerkt, dass der jeweilige Sensorhalter in den Abschnitten, in denen die FBG-Faser in x- und y-Richtung sensitive Kraftsensorbereiche aufweist, in axialer Richtung im Wesentlichen steif ist.

In ähnlicher Weise sind Ausführungsformen des vorgeschlagenen Katheters ausgestaltet, und auch im Hinblick auf den Katheter wird von einer Wiederholung dieser Merkmale Abstand genommen. Es wird aber ausdrücklich darauf hingewiesen, dass Katheter bzw. Katheteranordnungen aufgrund der vorliegenden Erfindung sich sinnvollerweise sowohl mit einem Kraftsensor mit im Wesentlichen rein axialer Empfindlichkeit als auch mit einer Kraftmesseinrichtung mit dreiachsigem Ansprechverhalten realisieren lassen. In jeden Fall ergibt sich eine Vereinfachung der zugehörigen Signalverarbeitung und, jedenfalls in zweckmäßigen Ausführungen, auch eine Vereinfachung der Sensorkonfiguration durch den möglichen Fortfall von Temperatursensoren bzw. -sensorbereichen.

Vorteile und Zweckmäßigkeiten der Erfindung ergeben sich im Übrigen aus der nachfolgenden Beschreibung von Ausführungsbeispielen anhand der Figuren. Von diesen zeigen:

Fig. 1        eine Prinzipskizze des Aufbaus einer FBG-Faser,

Fig. 2        eine schematische Darstellung zur Erläuterung der Erfindung,

Fig. 3A und 3B        eine perspektivische Detailansicht bzw. Längsschnittdarstellung zur Erläuterung einer Ausführungsform des erfindungsgemäßen Kraftsensors,

Fig. 4A und 4B        eine perspektivische Detailansicht bzw. Längsschnittdarstellung zur Erläuterung einer weiteren Ausführungsform des erfindungsgemäßen Kraftsensors,

Fig. 5A und 5B        eine perspektivische Darstellung bzw. Längsschnittdarstellung einer Ausführungsform der erfindungsgemäßen Kraftmesseinrichtung,

Fig. 6 und 6A        eine Längsschnittdarstellung bzw. Ausschnittdarstellung (Seitenansicht) einer weiteren Ausführungsform der erfindungsgemäßen Kraftmesseinrichtung,

Fig. 7          eine skizzenartige Darstellung einer erfindungsgemäßen Katheteranordnung.

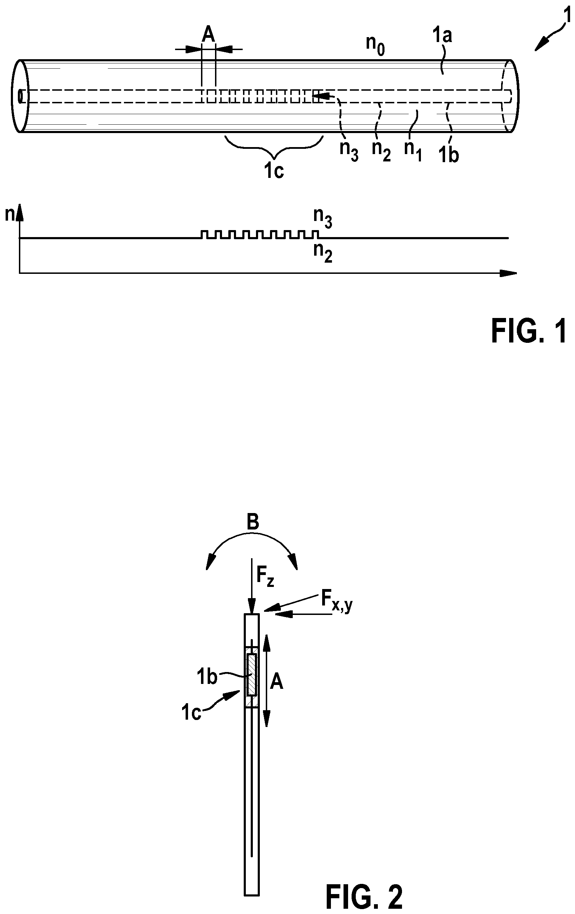

Fig. 1 zeigt im oberen Teil als schematische perspektivische Darstellung einen Abschnitt einer FBG-Faser 1, die eingebettet in einen Mantel 1a mit einem Brechungsindex $n_1$, einen Kern 1b hat, dessen Brechungsindex im Wesentlichen über die gesamte Längserstreckung $n_2$ ist. In einem Sensorabschnitt 1c der Faser 1 ist ein Bragg-Gitter ausgebildet, in dem Abschnitte mit dem Brechungsindex $n_2$ sich mit Abschnitten eines anderen Brechungsindex $n_3$ abwechseln, wie im unteren Teil der Figur verdeutlicht. Der Sensorabschnitt $1_c$ der Faser 1 kann unter anderem zur Erfassung von Spannungen bzw. äußeren Krafteinwirkungen oder Temperaturänderungen genutzt werden, wie aus dem Stand der Technik bekannt ist und daher hier nicht genauer beschrieben wird.

Grundsätzlich muss zur Berechnung eines 3D-Kraftvektors eine Kalibrierung des Sensorsystems stattfinden indem der Sensor nacheinander mit drei zueinander senkrechten Kräften (Fx, Fy, Fz) belastet wird.

In der Theorie berechnen sich die Dehnungen $\varepsilon_1$, $\varepsilon_2$ und $\varepsilon_3$ in den verteilten Sensorbereichen mit den Biegesteifigkeiten E x I (mit E-Modul des Materials und den entsprechenden Elementen der Biegesteifigkeit-Matrix), den Biegemomenten F x 1 (mit den entsprechenden Kraftkomponenten und dem jeweiligen Hebel 1 des Biegemomentes) und dem jeweiligen Abstands-Element $r_{ij}$ zur dehnungsneutralen Faser, sowie der Querschnittsfläche A, die der axial wirkenden Kraftkomponente entgegengesetzt wird, wie folgt:

$$\varepsilon_1 = \frac{F_x \cdot l_1}{E \cdot I_{xx1}} \cdot r_{11} + \frac{F_y \cdot l_1}{E \cdot I_{yy1}} \cdot r_{12} + \frac{F_z}{A \cdot E}$$

$$\varepsilon_2 = \frac{F_x \cdot l_2}{E \cdot I_{xx2}} \cdot r_{21} + \frac{F_y \cdot l_2}{E \cdot I_{yy2}} \cdot r_{22} + \frac{F_z}{A \cdot E} \tag{1}$$

$$\varepsilon_3 = \frac{F_x \cdot l_3}{E \cdot I_{xx3}} \cdot r_{31} + \frac{F_y \cdot l_3}{E \cdot I_{yy3}} \cdot r_{32} + \frac{F_z}{A \cdot E}$$

Mit drei Messungen, in denen drei zueinander senkrechte Kräfte verwendet werden, kann eine entsprechende Kalibrierungsmatrix berechnet werden:

$$\begin{pmatrix} C_{11} & C_{12} & C_{13} \\ C_{21} & C_{22} & C_{23} \\ C_{31} & C_{32} & C_{33} \end{pmatrix} = \begin{pmatrix} F_x & 0 & 0 \\ 0 & F_y & 0 \\ 0 & 0 & F_z \end{pmatrix} \cdot \begin{pmatrix} \varepsilon_{11} & \varepsilon_{12} & \varepsilon_{13} \\ \varepsilon_{21} & \varepsilon_{22} & \varepsilon_{23} \\ \varepsilon_{31} & \varepsilon_{32} & \varepsilon_{33} \end{pmatrix}^{-1} \tag{2}$$

Dabei müssen die Biegesteifigkeiten *EI* und Abstände zur neutralen Faser r in dem Sensorhalter in der Art konstruiert werden, dass sich drei linear unabhängige Gleichungen ergeben. So ist es möglich einen 3D-Kraftvektor aus gemessenen Dehnungen einer beliebigen Kraftrichtung mit Hilfe der Kalibrierungsmatrix zu berechnen:

$$\begin{pmatrix} F_x \\ F_y \\ F_z \end{pmatrix} = \begin{pmatrix} C_{11} & C_{12} & C_{13} \\ C_{21} & C_{22} & C_{23} \\ C_{31} & C_{32} & C_{33} \end{pmatrix} \cdot \begin{pmatrix} \varepsilon_1 \\ \varepsilon_2 \\ \varepsilon_3 \end{pmatrix} \tag{3}$$

Diese Kalibrierungsdaten werden optional im Katheter auf einem EEPROM, RFID oder einem 2D-Barcode gespeichert und von der Auswerteeinheit drahtlos, drahtgebunden oder beim 2D-Barcode optisch ausgelesen.

Fig. 2 zeigt schematisch, zur Verdeutlichung eines wesentlichen Aspekts der Erfindung, eine Seitenansicht eines in axialer Richtung (z-Richtung) sensitiven Kraftsensors, der sich bei einer in der xy-Ebene wirkenden Kraft versteift.

Für das Funktionsprinzip des rein z-sensitiven Sensors muss nach Gleichung (1) eine möglichst hohe Biegesteifigkeit Ixx und Iyy für Kräfte aus der xy-Richtung und ein möglichst geringer Widerstand für Kräfte aus der z-Richtung erreicht werden. Wie in Fig. 2 (als Bereich A) dargestellt, ist der Sensorbereich in z-Richtung weich und gibt bei einer Kraft $F_z$ leicht nach. Für Kräfte aus x- und y-Richtung (symbolisch dargestellt mit dem Doppelpfeil B) versteift sich der Sensor, so dass in diesen Richtungen ein erhöhtes Biegemoment vorhanden ist.

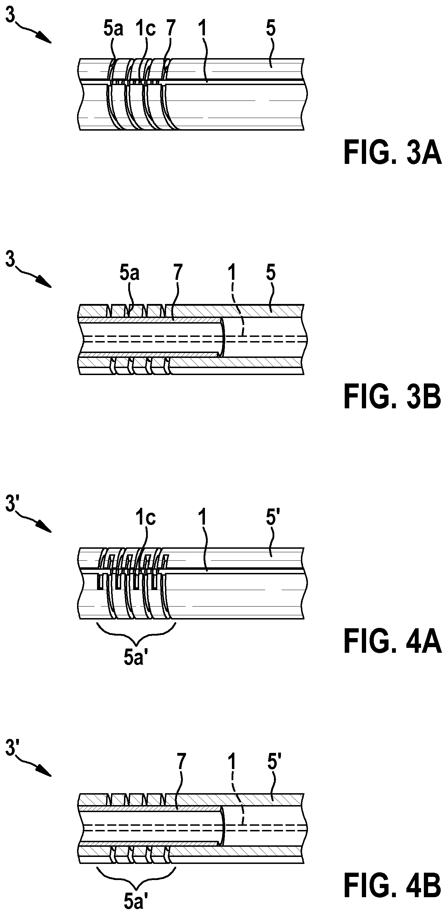

Fig. 3A und 3B zeigen in einer perspektivischen Ansicht bzw. Längsschnittdarstellung einen Abschnitt eines Kraftsensors 3 mit im Wesentlichen langgestreckt zylindrischer Gestalt, der als Kraftmesselement eine FBG-Faser 1 mit einem Sensorbereich 1c des in Fig. 1 gezeigten und weiter oben beschriebenen Typs umfasst. Die FBG-Faser 1 ist auf einem mit einer helixförmigen Nut 5a versehenen Außenrohr (Sensorhalter) 5 angebracht, in dem ein Innenrohr (Versteifungsteil) 7 in Gleitpassung zum Außenrohr 5 untergebracht ist.

Bei im Wesentlichen axial einwirkenden Kräften ist der Bereich des Außenrohrs 5, in dem die helixförmige Nut 5a eingearbeitet ist elastisch weich, so dass die axiale Kraftkomponente weitgehend unverfälscht in den Sensorbereich 1c eingeleitet wird. Senkrecht zur Achsenrichtung wirkenden Kraftkomponenten hingegen setzt das steife Innenrohr 7 ein hohes Biegemoment entgegen, so dass eine Biegeverformung des Sensorhalters und somit auch der darauf angebrachten FBG-Faser 1 weitestgehend unterbunden wird. Der Kraftsensor 3 ist somit sensitiv gegenüber in Achsenrichtung (z-Richtung) wirkenden Kräften, spricht aber im Wesentlichen nicht auf senkrecht hierzu angreifende Kräfte an.

Gleichartig funktioniert ein in Fig. 4A und 4B gezeigter Kraftsensor 3', der ganz ähnlich dem Sensor 3 nach Fig. 3A und 3B aufgebaut ist. Er unterscheidet sich von jenem nur dadurch, dass an die Stelle der helixförmigen Nut 5a im Außenrohr (Sensorhalter) 5' ein Abschnitt 5a' getreten ist, in dem alternierend kreissegmentförmige Einschnitte in gegenüberliegenden Umfangsbereichen des Sensorhalters vorgesehen sind, deren Enden sich unter dem Sensorbereich 1c der FBG-Faser 1 überlappen. Diese alternierenden, einander überlappenden Einschnitte verleihen dem Außenrohr (Sensorhalter) 5' ein ähnlich weichelastisches Ansprechverhalten wie die helixförmige Nut bei der vorgenannten Ausführung und gewährleisten damit die Empfindlichkeit des Kraftsensors 3' gegenüber axial einwirkenden Kräften, während das steife Innenrohr 7 für dessen Unempfindlichkeit gegenüber senkrecht zur Längsachse angreifenden Kräften sorgt.

Beide vorab beschriebenen Ausführungen des Kraftsensors sind grundsätzlich auch mit abweichend konfiguriertem Sensorhalter und/oder Versteifungselement realisierbar, etwa mit einem in seiner Gesamtheit als (Kunststoff-)Helix geformten Sensorhalter und einem drei-, vier- oder mehrkantigen Versteifungselement, und auch in einer Vertauschung der Anordnung von Halter- und Versteifungselement derart, dass der Sensorhalter innerhalb des Versteifungselementes platziert ist. In der Anwendung des Kraftsensors in einem Katheter kommt die oben beschriebene langgestreckt zylindrische Gestalt vorteilhaft zur Geltung, da sie günstig in einen flexiblen Katheterschlauch, eine Elektrodenleitung o. ä. integriert werden kann.

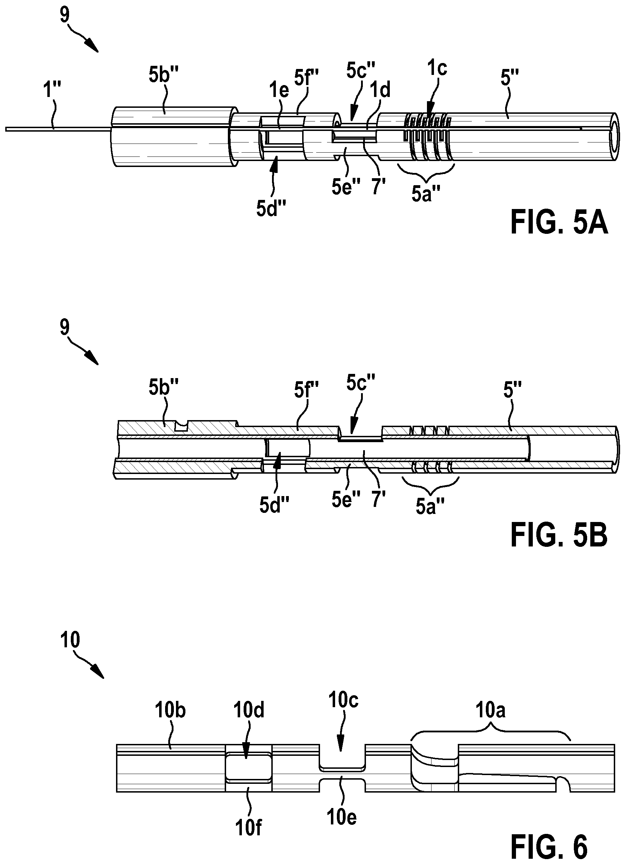

Die Figuren 5A und 5B zeigen in einer perspektivischen Ansicht bzw. Längsschnittdarstellung eine mehrachsig (dreiachsig) detektierende Kraftmesseinrichtung 9, die von dem oben beschriebenen Wirkprinzip des einachsig in Längsachsen-Richtung empfindlichen Kraftsensors und speziell der in Fig. 4A und 4B gezeigten Ausführung desselben Gebrauch macht. Soweit die Kraftmesseinrichtung 9 Teile oder Abschnitte enthält, die in anderen Figuren zu sehen und weiter oben beschrieben sind, werden in Fig. 5A und 5B die in den anderen Figuren verwendeten oder an jene angelehnte Bezugsziffern benutzt.

Ziffer 1' bezeichnet eine modifizierte FBG-Faser, auf der neben dem die Kraftwirkung in z-Richtung erfassenden Kraftsensorbereich 1c zwei weitere Kraftsensorbereiche 1d, 1e vorgesehen sind, auf denen - dank einer entsprechenden Konstruktion des Sensorhalters - in x- bzw. y-Richtung wirkende Kraftkomponenten erfasst werden. Der hier mit Ziffer 5" bezeichnete Sensorhalter (Außenrohr) ist in seinem in den Figuren rechts oben gezeigten Abschnitt praktisch identisch mit dem Sensorhalter 5' nach Fig. 4A und 4B, hat aber ein durch Vergrößerung des Durchmessers verstärktes gegenüberliegendes Ende 5b" und im mittleren Bereich zwei Paare kreissegmentförmige Ausnehmungen 5c" und 5d". Jedes dieser Paare umfasst zwei einander gegenüberliegende Ausnehmungen, zwischen denen jeweils zwei Stege 5e", 5f" stehen bleiben und die eine hohe Biegeelastizität bzw. eine niedriges Biegemoment jeweils in eine Vorzugsrichtung zur Folge haben. Da die Orientierung der Ausnehmungs-Paare 5c" und 5d" um 90° gegeneinander versetzt ist, ist auch die jeweilige Richtung höchster Biegsamkeit um 90° gegeneinander verdreht, so dass beide Ausnehmungs-Paare zusammen gewährleisten, dass die FGB-Faser 1' mit ihren den Ausnehmungen räumlich zugeordneten Kraftsensorbereichen 1d und 1e Kraftkomponenten innerhalb der xy-Ebene (also senkrecht zur Längsachse des Sensorhalters) detektieren kann.

Da auch das Innenrohr (Versteifungsteil), welches hier mit Ziffer 7' bezeichnet ist, am Ort der Ausnehmungs-Paare 5c" und 5d" selbst (nicht gesondert bezeichnete) Ausnehmungen aufweist, wirkt es an denjenigen Stellen, an denen es auf hohe Biegsamkeit ankommt, gleichfalls nicht versteifend und steht daher einer weitgehend unverfälschten Detektion von xy-Kraftkomponenten bzw. auf die FBG-Faser 1' einwirkenden Biegekräften nicht im Wege. Zusammen mit der durch den Kraftsensorbereich 1c realisierten axialen Empfindlichkeit ergibt sich insgesamt eine Kraftmesseinrichtung mit dem erwähnten dreiachsigen ("3D") Ansprechverhalten.

In diesem hier beschriebenen System kann jeder Sensorbereich bezüglich seiner Kraftrichtung individuell mit einer optimierten Sensitivität eingestellt werden. So kann der Dynamikbereich der FBG-Sensoren auch optimal ausgenutzt werden. Durch Ausnutzung des insgesamt zur Verfügung stehenden Dynamikbereiches bei mechanischer Belastung wird eine deutlich größere Sensitivität bezüglich einer Krafteinwirkung im Vergleich zu thermischen Einflüssen erreicht, weshalb eine Temperaturkompensation wegfallen kann. Damit lassen sich auch die Komplexität und die Kosten für ein solches System reduzieren.

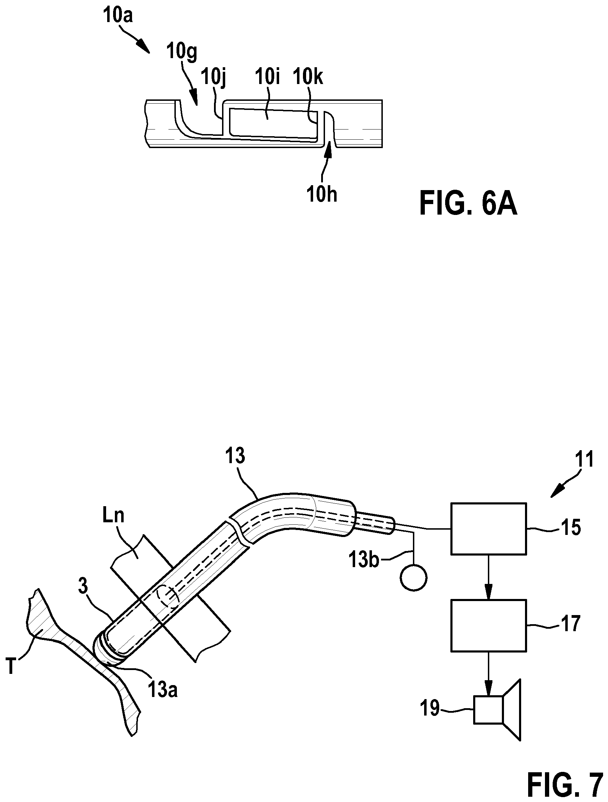

Fig. 6 zeigt in einer Längsschnittdarstellung einen einstückigen Sensorhalter 10 einer weiteren Ausführungsform der erfindungsgemäßen Kraftmesseinrichtung (dargestellt ohne FBG-Faser). Da der Sensorhalter 10 gewisse Ähnlichkeiten mit dem Sensorhalter (Außenrohr) 5" nach Fig. 5A und 5B aufweist, ist die Bezeichnungsweise der einzelnen Abschnitte an die in jenen Figuren angelehnt. Während der Aufbau desjenigen Teils, in dem bei angebrachter FBG-Faser deren x- und y-Kraftsensorbereiche angeordnet sind, weitgehend mit dem Aufbau des Sensorhalters 5" übereinstimmt, ist der Abschnitt 10a, der speziell zur Erfassung axial gerichteter Kräfte ausgebildet ist, anders gestaltet, um auf das bei der vorstehend beschriebenen Ausführung benötigte Versteifungsteil (Innenrohr) verzichten zu können.

Zur besseren Verdeutlichung der Konfiguration jenes Abschnitts ist er in Fig. 6A als Ausschnitt nochmals skizzenartig in einer Seitenansicht gezeigt. Es handelt sich um eine Ansicht in der Ebene, die durch die Längsachse und die Achse des Verlaufs der FBG-Faser (nicht gezeigt) aufgespannt wird. Wie dort zu erkennen ist, sind seine wesentlichen Merkmale zwei tiefe Einschnitte 10g, 10h im Sensorhalter, die von gegenüberliegenden Seiten eingebracht sind, und eine durchgehende Ausnehmung 10i, die unter einem Winkel von 90° zu beiden Ausnehmungen 10g, 10h ausgerichtet ist. Dazwischen bleiben schmale Stege 10j, 10k stehen, die zusammen mit den sich gegensinnig verjüngenden stehengebliebenen (nicht gesondert bezeichneten) Rohrabschnitten des Sensorhalters, die die Stege 10j, 10k miteinander verbinden, eine Art Parallelogramm bilden. Auf axial einwirkende Kräfte reagiert diese geometrische Konfiguration weich elastisch, während radial (in der xy-Ebene) angreifenden Kräften durch die Stege 10j, 10k ein hoher Widerstand entgegengesetzt wird.

Diese Konfiguration, die beispielsweise per Laser in einen Kunststofftubus eingeschnitten werden kann, ermöglicht einen konstruktiv einfachen und somit kostensparenden Aufbau des Sensorhalters, bei dem außerdem gewisse Nachteile der vorstehend beschriebenen zweiteiligen Ausführungen hinsichtlich der Messgenauigkeit vermieden werden können.

Fig. 7 zeigt skizzenartig eine Katheteranordnung 11, die einen Ablationskatheter 13 mit einer Spitzenelektrode 13a umfasst, in Kontakt mit einem Körpergewebsabschnitt T eines Patienten. Im Hinblick auf eine bestehende Perforationsgefahr des Körpergewebes T ist der Ablationskatheter 13 mit einer im distalen Endabschnitt eingeordetenen Kraftmesseinrichtung ausgestattet, die in der Figur (unter Bezugnahme auf die Figuren 3A und 3B) mit Ziffer 3 bezeichnet ist. Diese Kraftmesseinrichtung ist als einachsiger Kraftsensor ausgebildet, in den aus einem Gewebekontakt mit dem Gewebe T herrührende und im Wesentlichen axial wirkende Kräfte über die elastisch gelagerte Spitzenelektrode 13a eingetragen werden. Das proximale Ende der Kraftmesseinrichtung 3 stellt eine "kraftneutrale" Ebene Ln dar, an der ein Zugdraht 13b zur Steuerung des Katheters angreifen kann. Es sind allerdings auch Konstruktionen ohne derartigen Zugdraht möglich.

Ein Kraftsignal wird am Ende des Ablationskatheters abgegriffen und einer Signalverarbeitungseinheit 15 zugeführt. Jene ist ausgangsseitig mit einer Vergleichereinheit 17 verbunden, in der eine Vergleichswert-Verarbeitung des aufbereiteten Kraftsignals ausgeführt und bei Ermittlung eines gefährlich hohen Betrages der registrierten Axialkraft ein Ansteuersignal an eine (hier als Lautsprecher dargestellte) Warneinrichtung 19 ausgegeben wird. Der Arzt wird also aufgrund der Signale des Kraftsensors 3 über eine bestehende Perforationsgefahr informiert und kann entsprechend reagieren.

Die Ausführung der Erfindung ist nicht auf die oben beschriebenen Beispiele und hervorgehobenen Aspekte beschränkt, sondern kann ebenso in einer Vielzahl von Abwandlungen erfolgen, die im Rahmen fachmännischen Handelns liegen.

**Patentansprüche**

1. Faseroptischer Kraftsensor (3), umfassend eine in einem Sensorhalter (5) gehaltene FBG-Faser (1) mit einem Kraftsensorbereich (1c), wobei der Sensorhalter (5) in axialer Richtung abschnittsweise nachgiebig und biegeweich ist,
   **dadurch gekennzeichnet, dass**
   dem Sensorhalter (5) ein axial gegenüber dem Sensorhalter (5) verschiebliches, aber lateral unverschiebliches Versteifungsteil (7) zugeordnet ist, so dass lateral auf den Sensorhalter einwirkende Kräfte und Biegemomente unterdrückt werden und der Kraftsensorbereich (1c) ein im Wesentlichen einachsiges Ansprechverhalten zur Erfassung nur des Betrages einer axial in Faserrichtung wirkenden Kraft hat.

2. Faseroptischer Kraftsensor (3) nach Anspruch 1, wobei die FBG-Faser (1) zwei weitere Kraftsensorbereiche umfasst, und wobei die zwei weiteren Kraftsensorbereiche auf der FBG-Faser (1) winkelversetzt angeordnet sind, derart, dass sie zur Erfassung des Betrages und der Richtung der Projektion der einwirkenden Kraft auf die Ebene senkrecht zur Erstreckung der FBG-Faser (1) ausgebildet sind.

3. Faseroptischer Kraftsensor nach Anspruch 2, wobei die zwei weiteren Kraftsensorbereiche auf der FBG-Faser (1) um 90° winkelversetzt angeordnet sind.

4. Faseroptischer Kraftsensor (3) nach Anspruch 2 oder 3, wobei der Kraftsensorbereich (1c) und die zwei weiteren Kraftsensorbereiche in Längsrichtung der FBG-Faser (1) beabstandet auf dieser ausgebildet sind.

5. Faseroptischer Kraftsensor (3) nach einem der Ansprüche 2 bis 4, wobei der Sensorhalter (5) korrespondierend zur Position der Kraftsensorbereiche konstruktiv vorbestimmte mechanische Schwachstellen in Form einer spiralförmig verlaufenden Ausnehmung oder mehrerer kreissegmentförmiger Ausnehmungen in einem zylindrischen Haltergehäuse aufweist.

6. Katheter mit einem in einen distalen Abschnitt integrierten Faseroptischen Kraftsensor (3) nach einem der vorangehenden Ansprüche.

7. Katheter nach Anspruch 6, wobei der Katheter ein Ablationskatheter ist.

**Claims**

1. A fibre-optic force sensor (3), comprising an FBG fibre (1), which is mounted in a sensor holder (5) and has a force sensor region (1c), the sensor holder (5) being resilient and flexible in portions in the axial direction, **characterised in that** a stiffening part (7) that is displaceable axially relative to the sensor holder (5), but is not displaceable in a lateral direction relative to the sensor holder (5) is associated with the sensor holder (5), such that forces and bending moments acting laterally on the sensor holder are eliminated and the force sensor region (1c) has a substantially single-axis response behaviour to detect only the magnitude of a force acting axially in the fibre direction.

2. The fibre-optic force sensor (3) according to claim 1, wherein the FBG fibre (1) comprises two further force sensor regions, and wherein the two further force sensor regions are arranged on the FBG fibre (1) with an angular offset in such a way that they are designed to detect the magnitude and the direction of the projection of the effective force onto the plane perpendicular to the extent of the FBG fibre (1).

3. The fibre-optic force sensor according to claim 2, wherein the two further force sensor regions are arranged on the FBG fibre (1) with an angular offset of 90°.

4. The fibre-optic force sensor (3) according to claim 2 or 3, wherein the force sensor region (1c) and the two further force sensor regions are formed in a spaced manner on the FBG fibre (1) in the longitudinal direction thereof.

5. The fibre-optic force sensor (3) according to any one of claims 2 to 4, wherein the sensor holder (5) has, corresponding to the position of the force sensor regions, structurally predetermined mechanical weak points in the form of a spirally extending recess or a plurality of recesses shaped as segments of a circle in a cylindrical holder housing.

6. A catheter comprising a fibre-optic force sensor (3) according to any one of the preceding claims which is integrated in a distal portion of the catheter.

7. The catheter according to claim 6, wherein the catheter is an ablation catheter.

**Revendications**

1. Capteur de force à fibre optique (3) comprenant un réseau de Bragg inscrit dans la fibre, FBG de fibre, (1) maintenu dans un support de capteur (5) avec une région de capteur de force (1c), le support de capteur (5) s'accommodant par endroits en direction axiale et étant flexible, **caractérisé en ce** **qu'**une pièce de rigidification (7) coulissante axialement par rapport au support de capteur (5), mais non coulissante latéralement, est associée au support de capteur (5), de sorte que des forces et des moments de flexion agissant latéralement sur le support de capteur sont supprimés et que la région de capteur de force (1c) a une réactivité essentiellement dans un seul axe pour la détection uniquement de la contribution d'une force agissant axialement dans la direction de la fibre.

2. Capteur de force à fibre optique (3) selon la revendication 1, dans lequel le FGB de fibre (1) comprend deux autres régions de capteur de force et dans lequel les deux autres régions de capteur de force sont disposées décalées avec un angle sur le FGB de fibre (1) de telle manière qu'elles soient prévues pour la détection de la contribution et de la direction de la projection de la force agissant sur le plan perpendiculairement par rapport à l'extension du FGB de fibre (1).

3. Capteur de force à fibre optique (3) selon la revendication 2, dans lequel les deux autres régions de capteur de force sont disposées décalées avec un angle de 90° sur le FGB de fibre (1).

4. Capteur de force à fibre optique (3) selon la revendication 2 ou la revendication 3, dans lequel la région de capteur de force (1c) et les deux autres régions de capteur de force sont espacées dans la direction longitudinale du FGB de fibre (1) et sont formées sur celui-ci.

5. Capteur de force à fibre optique (3) selon l'une des revendications 2 à 4, dans lequel le support de capteur (5) présente des faiblesses mécaniques prédéterminées dans la construction correspondant à la position des régions de capteur de force sous la forme d'un évidement s'étendant en forme d'une spirale ou de plusieurs évidements en forme de segments circulaires dans un boitier de support cylindrique.

6. Cathéter doté d'un capteur de force à fibre optique (3) selon l'une des revendications précédentes intégré dans un segment distal.

7. Cathéter selon la revendication 6, où le cathéter est un cathéter d'ablation.

**FIG. 1**

**FIG. 2**

FIG. 3A

FIG. 3B

FIG. 4A

FIG. 4B

FIG. 5A

FIG. 5B

FIG. 6

**FIG. 6A**

**FIG. 7**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080285909 A1
- WO 2009138957 A2
- US 5399854 A
- WO 2009007857 A2
- WO 2008003307 A2
- US 20120220879 A1
- US 20090177095 A1

**In der Beschreibung aufgeführte Nicht-Patentliteratur**

- **A. OTHONOS ; K. KALLI.** Fiber Bragg Gratings: Fundamentels and Applications in Telecommunications and Sensing. *Artec House,* 1999